# EUROPEAN PATENT APPLICATION

(11) **EP 4 663 167 A1**
(43) Date of publication of application: **17.12.2025**
(21) Application number: 24753244.3
(22) Date of filing: 02.02.2024
(51) Int. Cl.: A61F 2/32

(54) **STEM FOR ARTIFICIAL JOINT**

(30) Priority: 08.02.2023 JP 2023017840
(71) Applicant: Kyocera Corporation, Kyoto-shi, Kyoto 612-8501 (JP)
(72) Inventor: IDA, Takao, Kyoto-shi, Kyoto 612-8501 (JP); WAKIYAMA, Miyo, Kyoto-shi, Kyoto 612-8501 (JP); SHIMOZONO, Takayoshi, Kyoto-shi, Kyoto 612-8501 (JP)
(74) Representative: TBK
(86) International application number: PCT/JP2024/003406
(87) International publication number: WO 2024/166801

(57) **Abstract**

Stable fixation to a bone. An artificial joint stem includes a base having a surface, and a first coating film located in a first region on the surface of the base. The first region includes a second region having a portion where the first coating film is not located. The second region includes a third region in which the first coating film is located and around which the first coating film is not located.

## Description

### TECHNICAL FIELD

The present disclosure relates to an artificial joint stem.

### BACKGROUND OF INVENTION

The use of a biological implant for the treatment of both bone injuries and diseases is constantly expanding with the growth of the active population and aging population. In such a situation, a known biological implant is provided with a coating from the viewpoint of antimicrobial properties, fixation to a bone, and the like.

For example, Patent Document 1 describes a coating for a medical implant, in which a part of the coating contains a bone-binding agent and an antimicrobial metal agent containing silver.

### CITATION LIST

### PATENT LITERATURE

Patent Document 1: JP 2011-512959 A

### SUMMARY

### PROBLEM TO BE SOLVED

There is a need for a biological implant that can be stably fixed to the bone.

### SOLUTION TO PROBLEM

The present disclosure provides an artificial joint stem including: a base having a surface; and a first coating film located in a first region on the surface of the base, in which: the first region includes a second region having a portion where the first coating film is not located; and the second region includes a third region in which the first coating film is located and around which the first coating film is not located.

### ADVANTAGEOUS EFFECT

According to the invention of the present disclosure, it is possible to provide an artificial joint stem that can be stably fixed to a bone.

### BRIEF DESCRIPTION OF THE DRAWINGS

FIG. 1 is a schematic view illustrating an artificial joint stem according to an embodiment.
FIG. 2 illustrates an overview of a second region of a base of the artificial joint stem according to the embodiment.
FIG. 3 is a view illustrating a cross section of the artificial joint stem according to the embodiment, taken along line IA-IA indicated by arrows in FIG. 1.
FIG. 4 illustrates an overview of the second region of the base in a manufacturing step for the artificial joint stem according to the embodiment.
FIG. 5 is a view illustrating a cross section of the artificial joint stem according to the embodiment, taken along line IB-IB indicated by arrows in FIG. 1.
FIG. 6 is a view illustrating a cross section of the artificial joint stem according to the embodiment, taken along line IC-IC indicated by arrows in FIG. 1.
FIG. 7 is a schematic view illustrating an artificial joint stem according to an embodiment.
FIG. 8 illustrates an overview of a second region of a base of the artificial joint stem according to the embodiment.
FIG. 9 is a view illustrating a cross section of the artificial joint stem according to the embodiment, taken along line VIII-VIII indicated by arrows in FIG. 7.
FIG. 10 is a schematic view illustrating an artificial joint stem according to an embodiment.
FIG. 11 is a schematic view illustrating an artificial joint stem according to an embodiment.
FIG. 12 is a view illustrating a cross section of the artificial joint stem according to the embodiment, taken along line IB-IB indicated by arrows in FIG. 1.
FIG. 13 is a view illustrating a cross section of the artificial joint stem according to the embodiment, taken along line IB-IB indicated by arrows in FIG. 1.
FIG. 14 is a flowchart illustrating a method of manufacturing an artificial joint stem according to an embodiment.
FIG. 15 is a schematic view illustrating an artificial hip joint according to an embodiment.
FIG. 16 is a schematic view illustrating an artificial joint stem according to an embodiment.
FIG. 17 is a schematic view illustrating an artificial joint stem according to an embodiment.
FIG. 18 is a schematic view illustrating an artificial joint stem according to an embodiment.
FIG. 19 is a schematic view illustrating an artificial joint stem according to an embodiment.
FIG. 20 is a schematic view illustrating a cross section of the artificial joint stem according to the embodiment.
FIG. 21 is a schematic view illustrating an artificial joint stem according to an embodiment.
FIG. 22 is a schematic view illustrating an artificial joint stem according to an embodiment.
FIG. 23 is a schematic view illustrating an artificial joint stem according to an embodiment.
FIG. 24 is a schematic view illustrating an artificial joint stem according to an embodiment.
FIG. 25 is a schematic view illustrating an artificial joint stem according to an embodiment.
FIG. 26 is a schematic view illustrating an artificial joint stem according to an embodiment.
FIG. 27 is a schematic view illustrating an artificial joint stem according to an embodiment.
FIG. 28 is a schematic view illustrating an artificial joint stem according to an embodiment.
FIG. 29 is a schematic view illustrating an artificial joint stem according to an embodiment.
FIG. 30 is a schematic view illustrating an artificial joint stem according to an embodiment.
FIG. 31 is a schematic view illustrating an artificial joint stem according to an embodiment.
FIG. 32 is a schematic view illustrating an artificial joint stem according to an embodiment.
FIG. 33 is a schematic view illustrating an artificial joint stem according to an embodiment.
FIG. 34 is a schematic view illustrating an artificial joint stem according to an embodiment.
FIG. 35 is a schematic view illustrating an artificial joint stem according to an embodiment.
FIG. 36 is a schematic view illustrating an artificial joint stem according to an embodiment.

### DESCRIPTION OF EMBODIMENTS

Hereinafter, one embodiment will be described in detail. Note that, unless otherwise specified herein, "A to B", which represents a numerical range, means "A or more and B or less".

### 1. Artificial Joint Stem

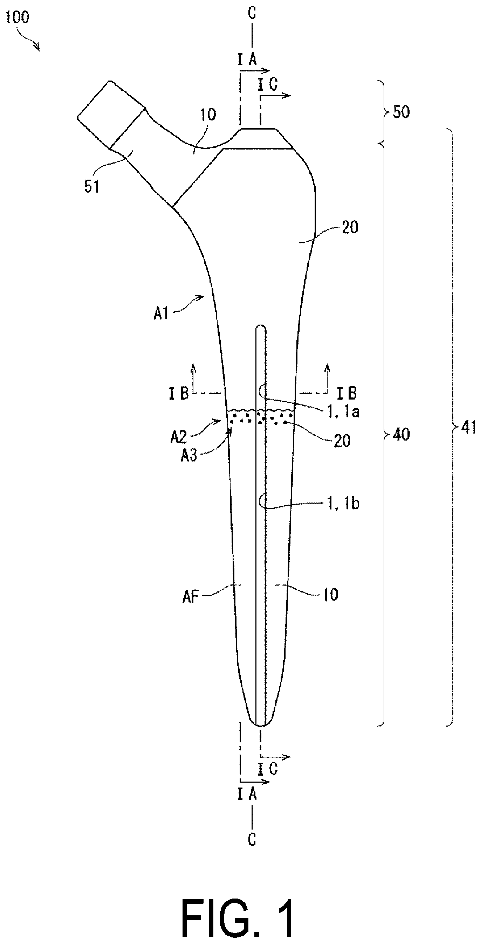

First, the configuration of an artificial joint stem 100 according to an embodiment will be described with reference to FIGs. 1 to 7. The artificial joint stem 100 includes a base 10 and a first coating film 20 located in a first region on the surface of the base 10. The term "surface" as used herein refers to the outermost side of each component. The "surface" includes not only a surface that can be visually recognized as an external appearance but also a surface on which a coating film such as the first coating film 20 is provided.

The artificial joint stem 100 includes an embedded portion 40 embedded in a bone and an exposed portion 50 exposed from the bone. Hereinafter, the side of the embedded portion 40 is referred to as a distal side, and the side of the exposed portion 50 is referred to as a proximal side. That is, the side embedded in the bone when the artificial joint stem 100 is used is defined as the distal side, and the opposite side is defined as the proximal side. The embedded portion 40 has a shape extending in a tapered shape from the proximal side toward the distal side.

In FIG. 1, of the embedded portion 40, the coating film 20 is formed in a first region A1 close to the exposed portion 50, and a distal region AF far from the exposed portion 50 is exposed from the first coating film 20. The distal region AF is mirror-finished. For example, only a partial region of the distal region AF may be mirror-finished. For example, the distal region AF may be processed by a chemical etching method or a blasting method.

A groove 1 having an elongated shape is located in the surface of the base 10. For example, the width of the groove 1 may be 2 to 5 mm. For example, the depth of the groove 1 may be 1 to 2.5 mm. This groove 1 contributes to facilitating the insertion of the artificial joint stem 100 into the bone. The number of the groove 1 may be one. The number of the groove 1 may be plural. The groove 1 includes a groove 1a located in the first region A1 where the first coating film 20 is disposed, and a groove 1b located in the distal region AF where the surface of the base 10 is exposed from the first coating film 20. That is, the first region A1 where the first coating film 20 is disposed includes at least a portion of the groove 1.

The length of the groove 1a is shorter than the length of the groove 1b. The groove 1a may be omitted. That is, the groove 1 may include at least the groove 1b. The groove 1 may include only the groove 1b. As a result, the adherence of the artificial joint stem 100 to the bone and the like can be adjusted. The ratio between the length of the groove 1a and the length of the groove 1b may be changed as appropriate depending on the size of the artificial joint stem 100.

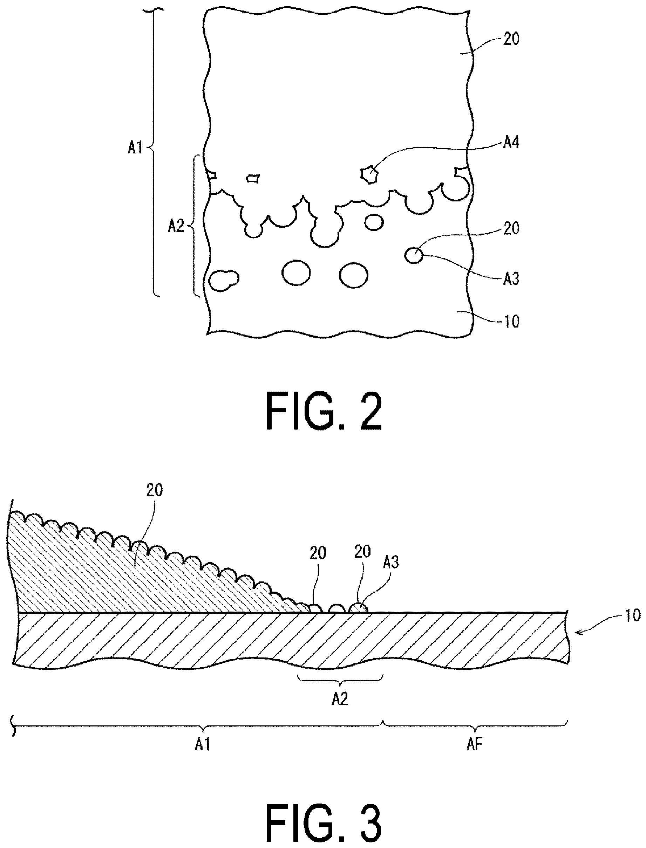

FIG. 2 illustrates an overview of a second region A2. FIG. 3 is a cross-sectional view taken along line IA-IA indicated by arrows in FIG. 1. The first coating film 20 is located in the first region A1 on the surface of the base 10. Thus, as illustrated in FIG. 3, the first region A1 is higher than the distal region AF where the first coating film 20 is not provided. Thus, when the artificial joint stem 100 is embedded in a bone, the first region A1 can mainly be brought into contact with the bone.

Here, the surface roughness in the first region A1 of the base 10 is higher than the surface roughness in the distal region AF of the base 10. Examples of surface roughness indices include arithmetic mean roughness Sa (ISO 25178). The surface roughness (Sa) of the first region A1 may be, for example, 10 to 80 µm. The surface roughness (Sa) of the first region A1 may be, for example, 20 to 80 µm. The surface roughness (Sa) of the first region A1 may be, for example, 30 to 70 µm. The surface roughness (Sa) of the base 10 in the distal region AF may be, for example, less than 1.0 µm.

Note that the surface roughness (Sa) of the first region A1 can be determined based on the measurement result of the entire first region A1. The surface roughness (Sa) of the distal region AF can be determined based on the measurement result of the entire distal region AF. For example, the first region A1 may include a portion having a surface roughness locally lower than the surface roughness of the distal region AF. For example, the distal region AF may include a portion having a surface roughness locally higher than the surface roughness of the first region A1.

The surface roughness (Sa) is only required to be measured, for example, by a stylus method or an optical method. The surface roughness (Sa) is only required to be measured in accordance with "ISO 25178", for example. The surface roughness (Sa) may be obtained from a measurement result in a partial region of the target region. Note that methods for measuring surface roughness are not limited to the above-described methods. Alternatively, the surface roughness (Sa) may be calculated from an image captured with an optical microscope or an electron microscope.

The surface of the first coating film 20 may be a rough surface. In this case, the region that mainly contacts the bone has a rough surface, improving the binding with the bone. While the first coating film 20 is formed by a thermal spraying method as described later in the present embodiment, this is not limiting. The first coating film 20 may have a porous structure.

The height of the first coating film 20 may have an upper limit of 1000 µm or less, for example. The height of the first coating film 20 may have an upper limit of 700 µm or less, for example. The surface roughness (Sa) of the first coating film 20 may be, for example, 10 to 80 µm. The surface roughness (Sa) of the first coating film 20 may be, for example, 20 to 80 µm. The surface roughness (Sa) of the first coating film 20 may be, for example, 30 to 70 µm.

As the material of the first coating film 20, the material exemplified later as the material of the base 10 can be used. For example, the first coating film 20 may be made of metal. The material of the first coating film 20 and the material of the base 10 may be the same. The material of the first coating film 20 and the material of the base 10 may be different. The material of the first coating film 20 may be, for example, a material containing pure titanium or a titanium alloy as a main component. Note that, in the present embodiment, the first coating film 20 is made of pure titanium.

The first region A1 includes a second region A2 having a portion where the first coating film 20 is not located. The second region A2 is adjacent to the distal region AF exposed from the first coating film 20. The second region A2 is a region at the end portion on the distal side in the first region A1.

The second region A2 is a third region A3 which is a region where the first coating film 20 is located and around which the first coating film 20 is not located. Accordingly, the first coating film 20 located in the third region A3 has an island shape surrounded by a region where the first coating film 20 is not located. When the artificial joint stem 100 having the above-described configuration is inserted into a bone, the third region A3 is considered to be a region having a low fixing property to the bone in the first region A1 and a region having a higher fixing property to the bone than the distal region AF. Therefore, when a force is applied to the artificial joint stem 100, the load applied to the bone in the boundary region between the first region A1 and the distal region AF can be dispersed. Therefore, the artificial joint stem 100 can be stably fixed to the bone.

The average height of the first coating film 20 in the second region A2 may be lower than the average height of the first coating film 20 in the entire first region A1. The second region A2 includes a plurality of third regions A3. The shapes of the plurality of third regions A3 may be different from each other. For example, the shape of the first coating film 20 in the third region A3 may be substantially hemispherical. For example, when the third region A3 is viewed from above, the shape of the first coating film 20 in the third region A3 may be a substantially circular shape or an elliptical shape.

As illustrated in FIG. 2, the second region A2 includes a fourth region A4 which is a region where the first coating film 20 is not located and around which the first coating film 20 is located. The fourth region A4 has a basin shape. When the artificial joint stem 100 having the above-described configuration is inserted into a bone, the fourth region A4 is considered to be a region having a low fixing property to the bone in the first region A1 and a region having a higher fixing property to the bone than the distal region AF. Therefore, when a force is applied to the artificial joint stem 100, the load applied to the bone in the boundary region between the first region A1 and the distal region AF can be dispersed. Therefore, the artificial joint stem 100 can be stably fixed to the bone.

The second region A2 includes a plurality of fourth regions A4. The shapes of the plurality of fourth regions A4 may be different from each other. The areas of the plurality of fourth regions A4 may be different from each other. The area of the fourth regions A4 in the entire second region A2 may be smaller than the area of the third regions A3 in the entire second region A2. The area of the fourth regions A4 in the entire second region A2 may be larger than the area of the third regions A3 in the entire second region A2.

As illustrated in FIG. 3, in the region of the first region A1 adjacent to the second region A2, the thickness of the first coating film 20 decreases toward the second region A2. Thus, the concentration of stress on the edge of the first coating film 20 can be reduced. In the region of the first region A1 adjacent to the second region A2, the surface roughness may decrease as the thickness of the first coating film 20 approaches the second region A2.

Here, a method of forming the first coating film 20 will be described. As described above, a thermal spraying layer 21 formed by spraying an atomized thermal spraying material or a semi-molten thermal spraying material is provided in the first region A1 on the surface of the base 10. At this time, the thickness of the thermal spraying layer 21 is adjusted by changing conditions such as the time for spraying the thermal spraying material, the temperature for spraying the thermal spraying material, or the pressure for spraying the thermal spraying material. Consequently, the thermal spraying layer 21 becomes thinner toward the edge.

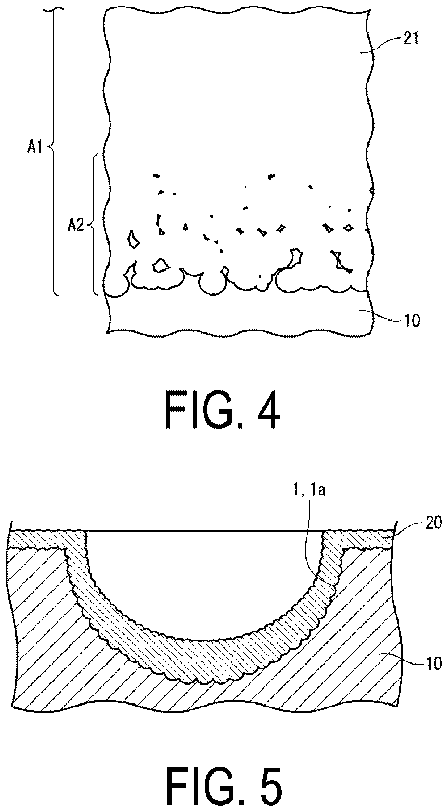

FIG. 4 illustrates an overview of the second region A2, illustrating a state in which the thermal spraying layer 21 has been formed. As illustrated in FIG. 4, in the second region A2, which is the edge, the thermal spraying layer 21 is thin, and there is a portion where the surface of the base 10 is exposed. In the second region A2, there are a plurality of portions where the surface of the base 10 is exposed.

Next, the surface roughness of the thermal spraying layer 21 is adjusted. Consequently, the first coating film 20 as illustrated in FIG. 2 is formed in the first region A1. The surface roughness can be adjusted by a mechanical processing method such as cutting, grinding, or sandblasting. The surface roughness can also be adjusted by a chemical processing method such as etching with an acid. The surface roughness can also be adjusted by a method in which a mechanical processing method and a chemical processing method are appropriately combined.

FIG. 5 is a cross-sectional view taken along line IB-IB indicated by arrows in FIG. 1. As described above, the groove 1a of the groove 1 is a part of the first region A1, and the first coating film 20 is formed in the groove 1a. As illustrated in FIG. 5, for an inside portion of the groove 1a and an outside portion of the groove 1a adjacent to each other in the first region A1, the thickness of the first coating film 20 in the inside portion of the groove 1a is greater than the thickness of the first coating film in the outside portion of the groove 1a. This can reduce the distance between the first coating film 20 in the inside portion of the groove 1a and the bone.

As illustrated in FIG. 5, the thickness of the first coating film 20 in the inside portion of the groove 1a increases toward a depth direction of the groove 1a. In the inside portion of the groove 1a, the thickness of the first coating film 20 at the deepest position of the groove 1a is greater than the thickness of the first coating film 20 at the shallowest position of the groove 1a. The thickness of the first coating film 20 in the inside portion of the groove 1a is greatest at the deepest position of the groove 1a.

For the inside portion of the groove 1a and the outside portion of the groove 1a adjacent to each other in the first region A1, the surface roughness of the first coating film 20 in the inside portion of the groove 1a is higher than the surface roughness of the first coating film 20 in the outside portion the groove 1a. This can increase the friction generated between the inside portion of the groove 1a and the bone.

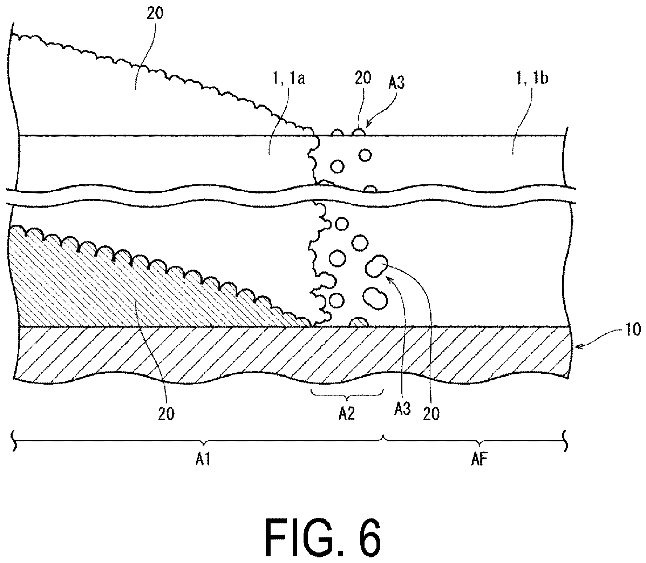

FIG. 6 is a cross-sectional view taken along line IC-IC indicated by arrows in FIG. 1. As illustrated in FIG. 6, also in the inside portion of the groove 1a, the first region A1 includes a second region A2 provided with a portion where the first coating film 20 is not formed. The second region A2 is adjacent to the distal region AF exposed from the first coating film 20. The second region A2 in the inside portion of the groove 1a is adjacent to the second region A2 in the outside portion of the groove 1a. The second region A2 in the inside portion of the groove 1a may not be adjacent to the second region A2 in the outside portion of the groove 1a.

Also, in the inside portion of the groove 1a, the second region A2 includes a third region A3 which is a region where the first coating film 20 is formed and around which the first coating film 20 is not formed. In the inside portion of the groove 1a, the second region A2 may not include the third region A3. The second region A2 may include the third region A3 only in the inside portion of the groove 1a.

As illustrated in FIG. 6, in the inside portion of the groove 1a, in the region of the first region A1 adjacent to the second region A2, the thickness of the first coating film 20 decreases toward the second region A2. That is, the thickness of the first coating film 20 in the inside portion of the groove 1a decreases from the proximal side toward the distal side. Thus, the concentration of stress on the edge of the first coating film 20 can be reduced when the internal surface of the groove 1a and the bone contact each other.

Referring to FIG. 6, in the second region A2, the area of the first coating film 20 located in the inside portion of the groove 1a is larger than the area of the first coating film 20 located in the outside portion of the groove 1a. That is, in the second region A2, the ratio of the surface area of the region where the first coating film 20 is not located to the surface area of the inside portion of the groove 1a is less than the ratio of the surface area of the region where the first coating film 20 is not located to the surface area of the outside portion of the groove 1a.

As illustrated in FIG. 5, in the first region A1, the surface roughness of the inside portion of the groove 1a is higher than the surface roughness of the outside portion of the groove 1a. In the distal region AF, the surface roughness of the inside portion of the groove 1b is higher than the surface roughness of the outside portion of the groove 1b. For example, the surface roughness (Sa) of the inside portion of the groove 1a may be 0.4 µm or more, and the surface roughness (Sa) of the outside portion of the groove 1a may be less than 0.05 µm. As illustrated in FIG. 5, the surface roughness of the inside portion of the groove 1 may increase toward the depth direction of the groove 1. In the distal region AF, the surface roughness of the inside portion of the groove 1 may be highest at the deepest portion of the groove 1.

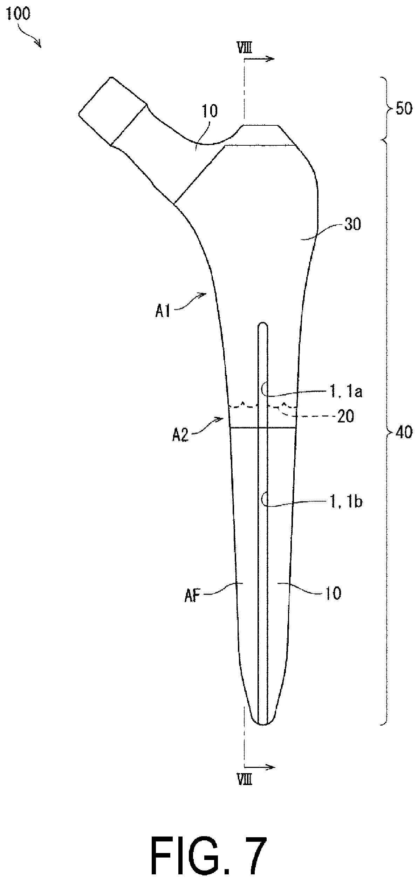

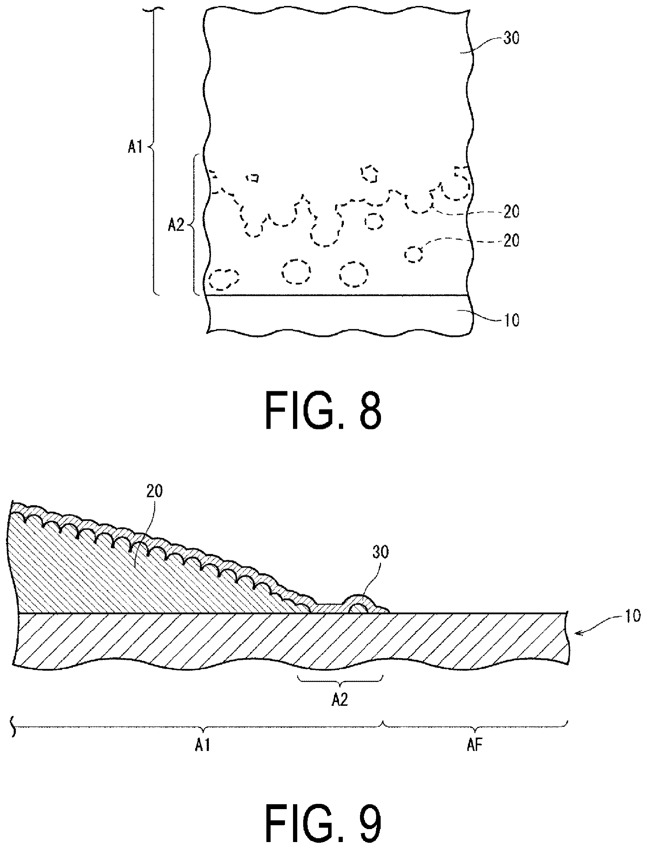

FIGs. 7 to 9 illustrate an example different from that in FIGs. 1 to 3. In the artificial joint stem 100 illustrated in FIGs. 7 to 9, a second coating film 30 is provided so as to cover the first coating film 20 in the first region A1 on the surface of the base 10. In the first region A1 on the surface of the base 10, the second coating film 30 is located on the first coating film 20.

The second coating film 30 is a member different from the first coating film 20. The second coating film 30 contains a calcium phosphate-based material and an antimicrobial material. The second coating film 30 may be configured not to contain the antimicrobial material. The calcium phosphate-based material is expected to improve adherence to the bone. The antimicrobial material is expected to reduce bacterial adhesion and growth.

Here, the surface of the second coating film 30 has a higher surface roughness than the surface of the base 10 in the distal region AF. Examples of surface roughness indices include arithmetic mean roughness Sa (ISO 25178). The surface roughness (Sa) of the second coating film 30 may be, for example, 10 to 80 µm. The surface roughness (Sa) of the second coating film 30 may be, for example, 20 to 80 µm. The surface roughness (Sa) of the second coating film 30 may be, for example, 30 to 70 µm.

Note that the surface roughness (Sa) of the second coating film 30 can be determined based on the measurement result of the entire first region A1. For example, the first region A1 may include a portion where the surface roughness of the second coating film 30 is locally lower than the surface roughness of the base 10 in the distal region AF. For example, the distal region AF may include a portion having a surface roughness locally higher than the surface roughness of the second coating film 30 in the first region A1.

Here, the second coating film 30 is disposed on a part of the surface of the base 10. That is, the other part of the surface of the base 10 is exposed from the second coating film 30. For example, it has been difficult to control the adherence of the artificial joint stem 100 to the bone when all of the surface of the artificial joint stem 100 is covered with a coating containing a bone-binding agent and an antimicrobial metal agent. In this case, removal of the artificial joint stem may become difficult when removal of the artificial joint stem is required after surgery. For example, the embedded portion 40 may become excessively adhered to the bone through the coating. When the base 10 includes the first region A1 covered with the second coating film 30 and the distal region AF exposed from the second coating film 30, excessive adhesion to the bone can be reduced. That is, both the adherence to the bone and the antimicrobial properties can be achieved.

At least part of the first region A1 is covered with the second coating film 30. That is, the entire surface of the first region A1 may be covered with the second coating film 30, or only part thereof may be covered with the second coating film 30. The surface of the second coating film 30 located in the first region A1 has a higher surface roughness than the surface of the base 10 in the distal region AF. Providing a region having a high surface roughness in this manner can improve the adherence to the bone. For example, the surface roughness of the second coating film 30 located in the first region A1 can be increased by forming a rough surface on the first coating film 20 in the first region A1 and covering the rough surface with the second coating film 30.

The surface roughness of the rough surface located in the first region A1 is higher than the surface roughness of the distal region AF. Thus, the surface roughness of the second coating film 30 located in the first region A1 is higher than the surface roughness of the distal region AF. The surface roughness (Sa) of the rough surface located in the first region A1 may be, for example, 10 to 80 µm. The surface roughness (Sa) of the rough surface located in the first region A1 may be, for example, 20 to 80 µm. The surface roughness (Sa) of the rough surface located in the first region A1 may be, for example, 30 to 70 µm. Note that the surface roughness of the rough surface can be measured, for example, by cutting the artificial joint stem 100 and observing the cut surface using an electron microscope or the like.

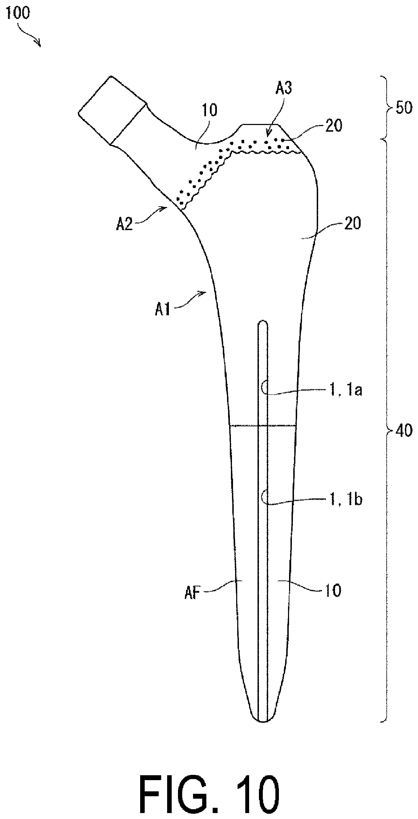

FIG. 10 illustrates an example different from that in FIGs. 1 to 3. In the artificial joint stem 100 illustrated in FIG. 10, the second region A2 is a region at the end portion on the proximal side in the first region A1. Therefore, the third region A3 and the fourth region A4 included in the second region A2 are also regions at the end portion on the proximal side in the first region A1. This allows the artificial joint stem to be stably fixed to the bone at the end portion on the proximal side in the first region A1.

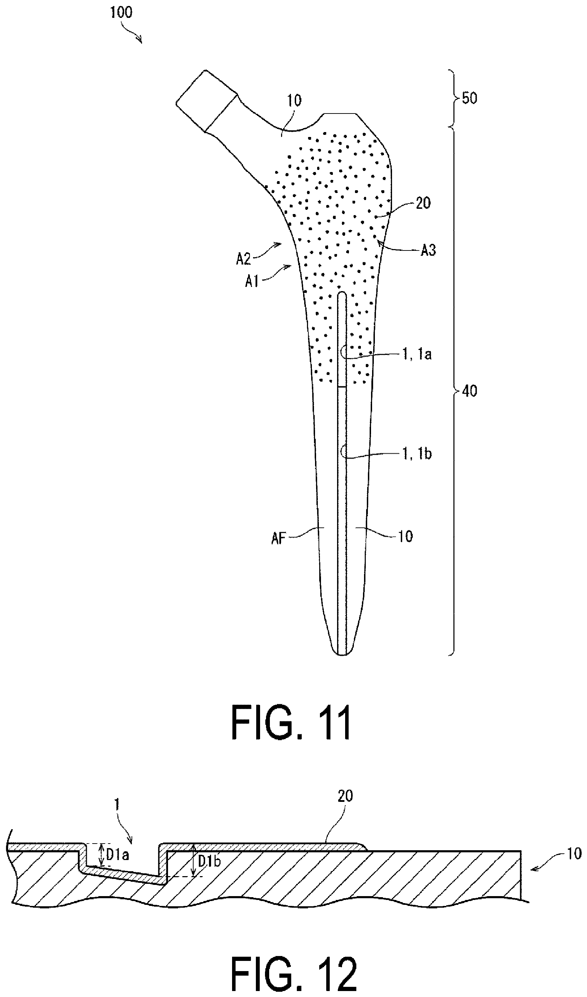

FIG. 11 illustrates an example different from that in FIGs. 1 to 3. In the artificial joint stem 100 illustrated in FIG. 11, the second region A2 is the entire region of the first region A1. This allows the artificial joint stem to be stably fixed to the bone at a portion of the embedded portion 40 on the proximal side.

FIG. 12 is a cross-sectional view taken along line IB-IB indicated by arrows in FIG. 1, illustrating an example different from that in FIG. 5. In FIG. 12, a depth D1a of the groove 1 at a first end portion in the width direction of the groove 1 is less than a depth D1b at a second end portion. The cross-sectional shape of the groove 1 may be a rectangular shape. In this case, the depth D1a of the groove 1 at the first end portion is equal to the depth D1b at the second end portion. The cross-sectional shape of the groove 1 may be a right triangle shape. In this case, the depth D1a of the groove 1 at the first end portion is zero.

In this manner, the shape of the groove 1 is not particularly limited. For example, the groove 1 may be a recess having a circular, polygonal, or indeterminate form. The artificial joint stem 100 in which such a recess is formed is also included in the present disclosure. The opening area of the above recess can be calculated by using image analysis software or the like.

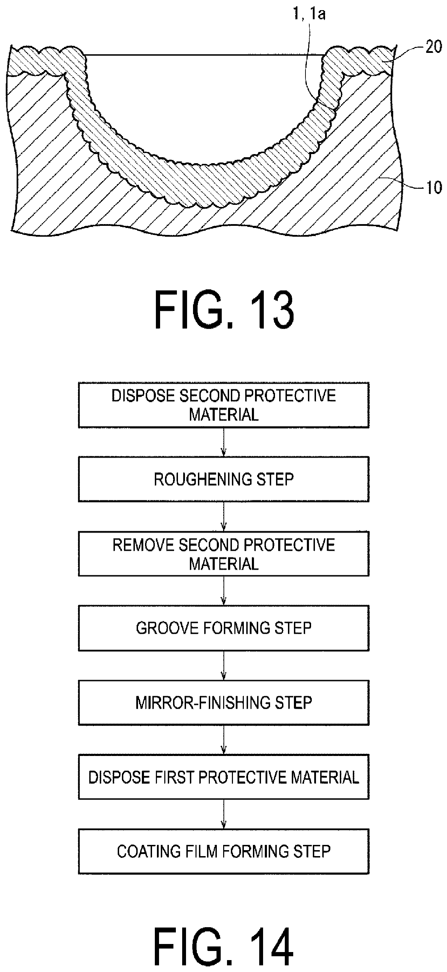

FIG. 13 is a cross-sectional view taken along line IB-IB indicated by arrows in FIG. 1, illustrating an example different from that in FIG. 5. In FIG. 13, in the first region A1, the surface roughness of the inside portion of the groove 1a is lower than the surface roughness of the outside portion of the groove 1a. As illustrated in FIGs. 5 and 13, for the inside portion of the groove 1 and the outside portion of the groove 1 adjacent to each other, the surface roughness of the inside portion of the groove 1 may be different from the surface roughness of the outside portion of the groove 1.

As the material of the base 10, for example, a metal, ceramic, or plastic may be used. When a metal is used as the material of the base 10, it is possible to use, for example, stainless steel alloys, cobalt chromium alloys, titanium, titanium alloys, and the like as the material of the base 10. When a titanium alloy is used as the material of the base 10, it is possible to use, for example, an alloy that can be made by adding at least one selected from the group consisting of aluminum, tin, zirconium, molybdenum, nickel, palladium, tantalum, niobium, vanadium, platinum, and the like to titanium as the material of the base 10.

When a ceramic is used as the material of the base 10, it is possible to use, for example, alumina, zirconia, an alumina-zirconia composite ceramic, and the like as the material of the base 10. When a plastic is used as the material of the base 10, it is possible to use, for example, polyethylene, fluorine-based resin, epoxy resin, polyetheretherketone (PEEK) resin, Bakelite, and the like as the material of the base 10. Note that, in the present embodiment, the base 10 is made of a titanium alloy.

The shape of the base 10 may be substantially rod-shaped, for example, but may be appropriately changed according to the shape of the artificial joint to be applied.

As described above, the base 10 may include the embedded portion 40 to be embedded in a bone and the exposed portion 50 to be exposed from the bone. The femur is an example of the bone. The embedded portion 40 may include at least part of the first region A1. In other words, the second coating film 30 may be formed on part of the peripheral wall of the embedded portion 40. Thus, the embedded portion 40, which can actually come into contact with the bone, can exhibit desired adherence and antimicrobial properties.

The base 10 as described above may include a body portion 41 that includes a surface including the first region A1 and the distal region AF, and a neck portion 51 connected to the end portion on the proximal side of the body portion 41. The body portion 41 may be embedded in a femur portion. The neck portion 51 is exposed from the femur, provided with a bone head, and may be placed in an acetabular cup that constitutes a pair with the artificial joint stem.

The body portion 41 has a center axis C extending along the groove 1. The body portion 41 includes an end surface on the proximal side disposed away from the center axis C, and the neck portion 51 is connected to the end surface on the proximal side. The neck portion 51 is smaller in width than the body portion 41 (the end surface on the proximal side). In other words, the neck portion 51 is a protruding portion 51 protruding from the body portion 41 in an oblique direction inclined from the center axis C.

The second coating film 30 contains a calcium phosphate-based material and an antimicrobial material. Examples of the calcium phosphate-based material include one or a mixture of two or more types selected from the group consisting of hydroxyapatite, α-tertiary calcium phosphate, β-tertiary calcium phosphate, quaternary calcium phosphate, octacalcium phosphate, and calcium phosphate-based glass. As the antimicrobial material, for example, a natural antimicrobial agent, an organic antimicrobial agent, or an inorganic antimicrobial agent can be used. As the natural antibacterial agent, for example, hinokitiol can be used. As the organic antibacterial agent, for example, benzalkonium chloride can be used. As the inorganic antibacterial agent, for example, a metal can be used. When a metal is used as the inorganic antimicrobial agent, it is possible to use, for example, silver, copper, zinc, and the like as the inorganic antimicrobial agent.

In addition to the calcium phosphate-based material and the antimicrobial material, the second coating film 30 may contain a glass ceramic, and may further contain an antimicrobial agent such as penicillin and vancomycin.

The concentration of the antimicrobial material in the second coating film 30 may be, for example, 0.05 wt% to 3.00 wt%. The concentration of the antimicrobial material in the second coating film 30 may be, for example, 0.05 wt% to 2.50 wt%. The concentration of the antimicrobial material in the second coating film 30 may be, for example, 0.05 wt% to 1.00 wt%. The concentration of the antimicrobial material in the second coating film 30 may be, for example, 0.1 wt% to 1.00 wt%. When the concentration of the antimicrobial material is 0.05 wt% or more, sufficient antimicrobial properties can be achieved. When the concentration of the antimicrobial material is 3.00 wt% or less, the impact on living tissue can be reduced.

There may be a concentration gradient of the antimicrobial material in the second coating film 30. For example, the concentration of the antimicrobial material contained in the end portion on the proximal side of the second coating film 30 may be greater than the concentration of the antimicrobial material contained in the end portion on the distal side of the second coating film 30. Thus, invasion of bacteria from the proximal side of the second coating film 30 can be reduced. The antimicrobial material may be contained only in the end portion on the proximal side of the second coating film 30.

The second coating film 30 may be disposed on the first coating film 20. As described above, the first coating film 20 may be mainly in contact with the bone. The presence of the second coating film 30 on the first coating film 20 can further improve the adherence to the bone and the antimicrobial properties.

The height of the first coating film 20 may be greater than the thickness of the second coating film 30. Thus, since the region where the first coating film 20 is formed is higher than the region where only the second coating film 30 is formed, the region where the first coating film 20 is formed can mainly be brought into contact with the bone. The thickness of the second coating film 30 may be, for example, less than 200 µm. The thickness of the second coating film 30 may be, for example, less than 100 µm. The thickness of the second coating film 30 may be, for example, less than 50 µm. The thickness of the second coating film 30 may be, for example, 5 µm or more.

At least part of the second region A2 may be covered with the second coating film 30. That is, the entire surface of the second region A2 may be covered with the second coating film 30, or only part thereof may be covered with the second coating film 30.

The second coating film 30 may be formed so as to cover the first coating film 20. This can avoid exposure of the first coating film 20 from the second coating film 30. As a result, bacterial growth can be further reduced.

The distal region AF is exposed from the second coating film 30. A region where the second coating film 30 is disposed and a region exposed from the second coating film 30 can be distinguished from each other by an elemental analysis of the surface of each region. The elemental analysis can be performed, for example, by mapping the surface elements with an energy dispersive X-ray spectrometry apparatus, which is an accessory for a general scanning electron microscope. Surface analysis methods such as X-ray photoelectron spectroscopy, Auger electron spectroscopy, and secondary ion mass spectrometry may also be used. The element may be confirmed by chemical analysis of a sample obtained by mechanically scraping off the surface of each region. For example, on at least part of the surface of the first region A1 where the second coating film 30 is disposed, phosphorus, calcium, antimicrobial components, or the like are detected. On the surface of the distal region AF, elements constituting the base 10 are detected, and phosphorus, calcium, antimicrobial components, or the like are not detected, or are detected at a noise level or lower.

The surface of the second coating film 30 located in the second region A2 may have a lower surface roughness than the surface of the second coating film 30 located in the first region A1 other than the second region A2. Accordingly, the adherence to the bone and the antimicrobial properties can be sufficiently achieved in the first region A1 other than the second region A2. Excessive adhesion to the bone can be reduced in the second region A2.

The surface roughness (Sa) of the second coating film 30 located in the first region A1 other than the second region A2 may be, for example, 10 to 80 µm. The surface roughness (Sa) of the second coating film 30 located in the first region A1 other than the second region A2 may be, for example, 20 to 80 µm. The surface roughness (Sa) of the second coating film 30 located in the first region A1 other than the second region A2 may be, for example, 30 to 70 µm. The surface roughness (Sa) of the second coating film 30 located in the second region A2 may be, for example, 0.1 to 10 µm.

The surface of the second coating film 30 located in the second region A2 may have a higher surface roughness than the surface of the base 10 in the distal region AF. Accordingly, in the second region A2, the adhesion between the second coating film 30 and the base 10 can be improved, and thus the adherence to the bone and the antimicrobial properties can be sufficiently achieved. Excessive adhesion to the bone can be reduced in the distal region AF.

The surface roughness of the base 10 in the second region A2 may be higher than the surface roughness of the base 10 in the distal region AF. Accordingly, in the second region A2, the adhesion between the second coating film 30 and the base 10 can be improved, and thus the peeling of the second coating film 30 can be reduced. The surface roughness (Sa) of the base 10 in the second region A2 may be, for example, 0.1 µm or more and less than 10 µm. The surface roughness (Sa) of the base 10 in the second region A2 may be, for example, less than 2.0 µm.

At least part of the second coating film 30 may be disposed in a boundary region 60 including the boundary between the embedded portion 40 and the exposed portion 50. That is, the second coating film 30 may be disposed in a region of the embedded portion 40 near the exposed portion 50. Thus, invasion of bacteria from the exposed portion 50 side can be reduced.

The second coating film 30 disposed in the boundary region 60 may be disposed only in the embedded portion 40. That is, the second coating film 30 need not be disposed in the exposed portion 50. Thus, irritation to the soft tissue that may come in contact with the exposed portion 50 can be reduced.

For example, the artificial joint stem 100 may carry iodine or a compound containing iodine on the entire surface thereof. For example, the artificial joint stem 100 may carry iodine or a compound containing iodine on a part thereof. For example, the artificial joint stem 100 may carry iodine or a compound containing iodine at least in the first region A1. For example, the artificial joint stem 100 may carry iodine or a compound containing iodine at least in the distal region AF. For example, the artificial joint stem 100 may carry iodine or a compound containing iodine only in the distal region AF. A known method may be adopted to cause the artificial joint stem 100 to carry iodine or a compound containing iodine. For example, the artificial joint stem 100 may be caused to carry iodine or a compound containing iodine by anodizing the surface of the artificial joint stem 100.

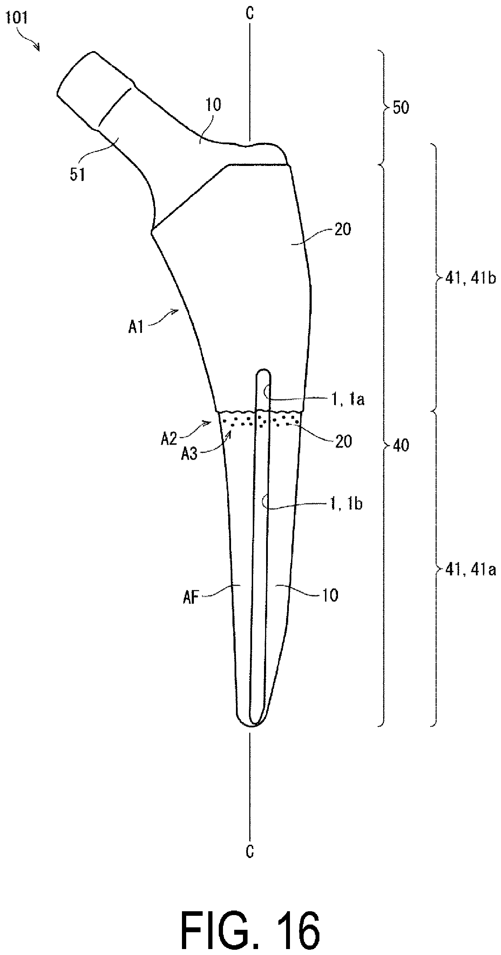

The artificial joint stem according to the present disclosure also includes an artificial joint stem 101 illustrated in FIG. 16. For example, the body portion 41 may include a distal portion 41a having the center axis C extending along the groove 1, and a proximal portion 41b extending toward the proximal side continuously from the distal portion 41a and shaped to curve such that the center of the proximal portion 41b departs further away from the center axis C as the proximal portion 41b extends toward the proximal side. The proximal portion 41b includes an end surface on the proximal side disposed away from the center axis C, and the neck portion 51 may be connected to the end surface on the proximal side.

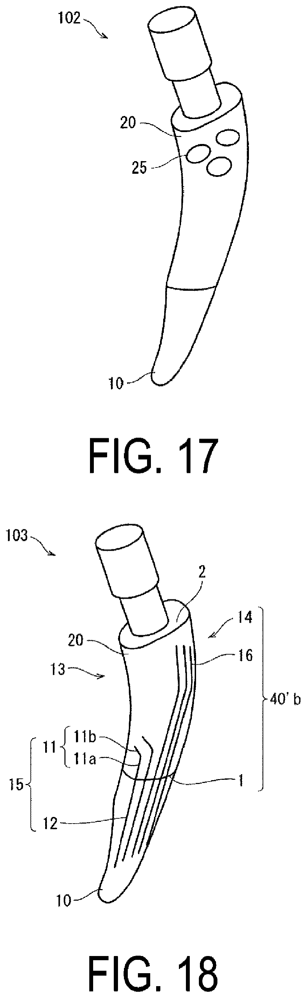

The artificial joint stem according to the present disclosure also includes an artificial joint stem 102 illustrated in FIG. 17. For example, a recessed portion 25 including an opening in the surface of the coating film 20 may be provided. The opening area of the recessed portion 25 located on the upper end portion side of the coating film 20 may be greater than the opening area of the recessed portion 25 located on the lower end portion side of the coating film 20. The recessed portion 25 may be provided only at the upper end portion of the coating film 20.

The artificial joint stem according to the present disclosure also includes an artificial joint stem 103 illustrated in FIG. 18. For example, the base 10 may include a groove. The groove may be disposed to straddle the region where the coating film 20 is disposed and the region exposed from the coating film 20. The groove may extend to the upper end portion of the coating film 20. The surface roughness in the groove located on the rough surface may be lower than the surface roughness of the base 10 located on the rough surface.

The base 10 may include an inner side portion 13 that curves concavely and an outer side portion 14 that curves convexly. Here, the groove extends in the vertical direction, and the upper end of the groove may be located at the curved portion. At a contraction portion 40'b, the groove may be bent toward either the inner side portion 13 or the outer side portion 14. For example, the groove may be bent at the contraction portion 40'b toward the inner side portion 13. The depth of the lower end portion of the groove may be less than the depth of the upper end portion of the groove. The width of the lower end portion of the groove may be less than the width of the upper end portion of the groove. A first end portion of the groove may be exposed from the coating film 20, and a second end portion of the groove may be located at the contraction portion 40'b.

The groove located in the region where the coating film 20 is disposed is defined as a first groove 11, and the groove located in the region where the surface of the base 10 is exposed from the coating film 20 is defined as a second groove 12. The first groove 11 may be connected to the second groove 12. That is, the first groove 11 and the second groove 12 may be formed as a series of grooves. The first groove 11 may not be connected to the second groove 12. That is, the first groove 11 and the second groove 12 may be formed as different grooves. The upper end portion of the first groove 11 may be bent toward the inner side portion 13. In other words, the first groove 11 may include a first portion 11a extending in the vertical direction of the base 10 and a second portion 1b connected to the first portion 11a and having a component along the width direction of the base 10. The depth of the second groove 12 may be less than the depth of the first groove 11.

The base 10 may include a plurality of grooves. The base 10 may also include a first groove set 15 that includes the first groove 11 and the second groove 12 connected to each other, and a second groove set 16 that includes the first groove 11 and the second groove 12 connected to each other with the first groove 11 extending toward the upper end portion of the coating film 20 further than the first groove set 15.

In addition, the base 10 may include a plurality of first groove sets 15. The plurality of first groove sets 15 may be arranged in the width direction of the base 10. Of the plurality of first groove sets 15, the first groove set 15 located on the outer side portion 14 side may be located above the first groove set 15 located on the inner side portion 13 side.

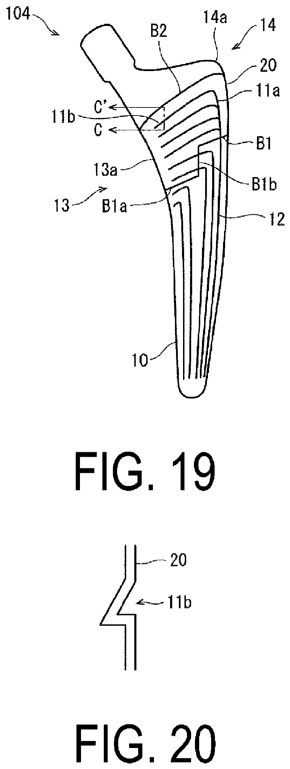

The artificial joint stem according to the present disclosure also includes an artificial joint stem 104 illustrated in FIG. 19. For example, the base 10 may include a plurality of grooves, and a groove located at the upper portion of the base 10 may be wider than a groove located at the lower portion of the base 10. The grooves may have a component along the width direction of the base 10. FIG. 20 illustrates a C-C' cross section of FIG. 19. As illustrated in FIG. 20, the groove along the width direction of the base 10 may become shallower toward the upper side.

The lower edge (first boundary line B1) of the coating film 20 may intersect the linear portion of the groove. Here, the first boundary line B1 may diagonally intersect the linear portion of the groove. That is, the first boundary line B1 need not be orthogonal to the linear portion of the groove.

The first boundary line B1 may include a first portion B1a extending in a direction intersecting the groove and a second portion B1b extending in a direction along the groove. The second portion B1b may be separated from the groove. That is, the second portion B1b need not be in contact with the groove. The upper edge (second boundary line B2) of the coating film 20 also may include a portion extending in a direction intersecting the groove and a portion extending in a direction along the groove.

The first groove 11 and the second groove 12 may be connected, and the first groove 11 may include a first portion 11a extending in the vertical direction of the base 10 and a second portion 11b connected to the first portion 11a and having a component along the width direction of the base 10. As illustrated in FIG. 19, the second boundary line B2 may extend in a direction along the second portion 11b of the first groove 111.

As illustrated in FIG. 19, the second boundary line B2 may be disposed so as to incline upward from the inner side portion 13 toward the outer side portion 14. As illustrated in FIG. 19, the first boundary line B1 may be located below an apex 13a of the recessed portion at the inner side portion 13. The second boundary line B2 may be located below an apex 14a of the protruding portion at the outer side portion 14. Alternatively, the first boundary line B1 may be located above the apex 14a of the protruding portion at the outer side portion 14.

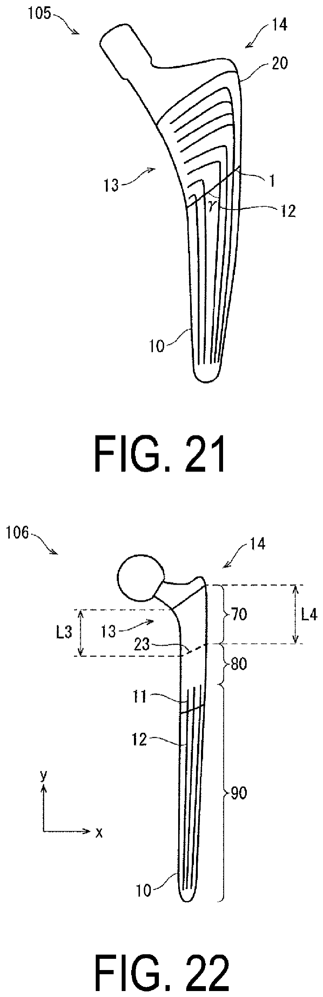

The artificial joint stem according to the present disclosure also includes an artificial joint stem 105 illustrated in FIG. 21. For example, the boundary line defined by the presence or absence of the coating film 20 may intersect the groove with an acute angle. In FIG. 21, among the angles formed by the first boundary line B1 and the second groove 12, an angle γ on the inner side portion 13 side is an acute angle.

The artificial joint stem according to the present disclosure also includes an artificial joint stem 106 illustrated in FIG. 22. For example, the base 10 includes, from top to bottom, a rough-surface region 70, a non-rough-surface region 80, and a groove region 90. A rough surface is disposed in the rough-surface region 70. The non-rough-surface region 80 is a region in which no rough surface is disposed. Grooves are disposed in the groove region 90. For example, the rough-surface region 70 may be a region in which a rough surface is disposed but no grooves are disposed. The non-rough-surface region 80 may be a region in which neither a rough surface nor a groove is disposed. The groove region 90 may be a region in which a rough surface is not disposed but grooves are disposed.

The coating film 20 may cover at least one selected from the group consisting of the rough-surface region 70, the non-rough-surface region 80 and the groove region 90. The area of the rough-surface region 70 may be smaller than the area of the non-rough-surface region 80. In the width direction of the base 10, the length of the rough-surface region 70 may be greater than the length of the non-rough-surface region 80. A boundary line 23 between the rough-surface region 70 and the non-rough-surface region 80 may be inclined upward from the inner side portion 13 toward the outer side portion 14. A length L3 on the inner side portion 13 side of the rough-surface region 70 may be less than a length L4 on the outer side portion 14 side of the rough-surface region 70. Here, the length L3 represents a difference in the Y coordinate between a point where the Y coordinate is maximum and a point where the Y coordinate is minimum, on the inner side portion 13 side of the rough-surface region 70. The length L4 represents a difference in the Y coordinate between a point where the Y coordinate is maximum and a point where the Y coordinate is minimum, on the outer side portion 14 side of the rough-surface region 70.

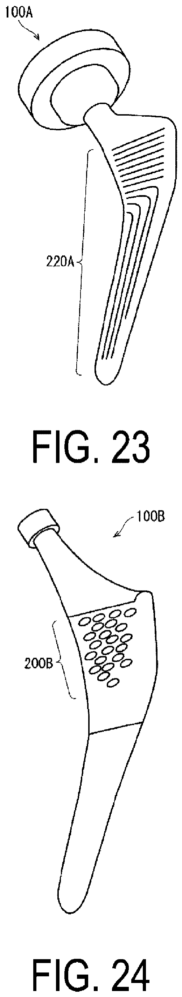

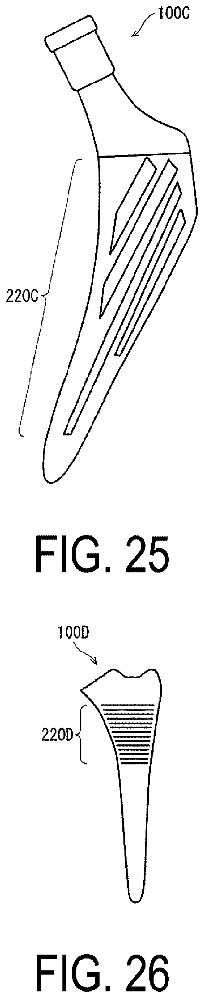

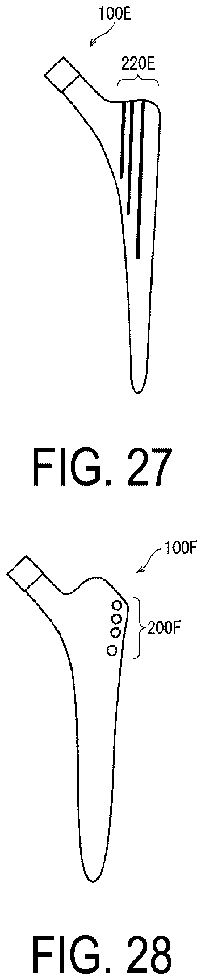

The artificial joint stem according to the present disclosure also includes artificial joint stems 100A to 100F illustrated in FIGs. 23 to 28. For example, a groove 220A may be provided as in the artificial joint stem 100A. For example, a hole 200B may be provided as in the artificial joint stem 100B. For example, a groove 220C may be provided as in the artificial joint stem 100C. For example, a groove 220D may be provided as in the artificial joint stem 100D. For example, a groove 220E may be provided as in the artificial joint stem 100E. For example, a hole 200F may be provided as in the artificial joint stem 100F. In the above example, the exposed region is provided at the upper and lower ends of the coating film 20, but as illustrated in FIG. 19, the exposed region may be located only on the upper side of the coating film 20.

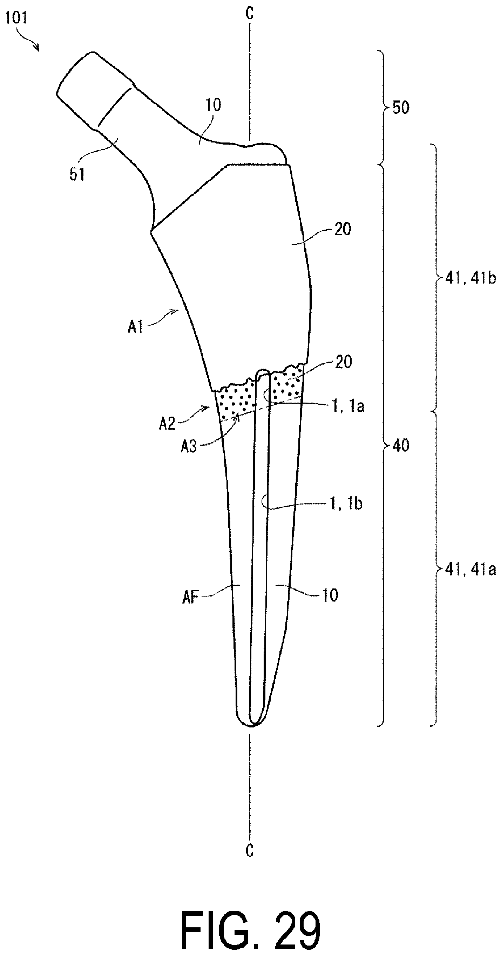

FIG. 29 illustrates an example different from that in FIG. 16. In the artificial joint stem 101 illustrated in FIG. 16, the second region A2 is a region extending along a direction perpendicular to the center axis C extending along the groove 1. In the artificial joint stem 101 illustrated in FIG. 29, the second region A2 is a region extending along an oblique direction with respect to the center axis C extending along the groove 1. In FIG. 29 and the subsequent drawings, the boundary between the second region A2 and the distal region AF is indicated by a dashed-dotted line.

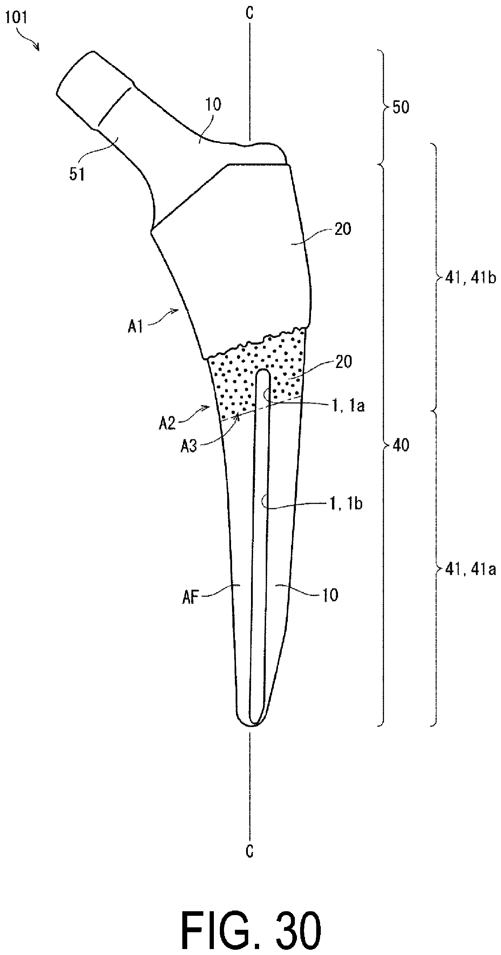

FIG. 30 illustrates an example different from that in FIG. 16. In the artificial joint stem 101 illustrated in FIG. 30, the second region A2 is a region extending along an oblique direction with respect to the center axis C extending along the groove 1. The end portion of the second region A2 on the proximal side is on the proximal side with respect to the groove 1a.

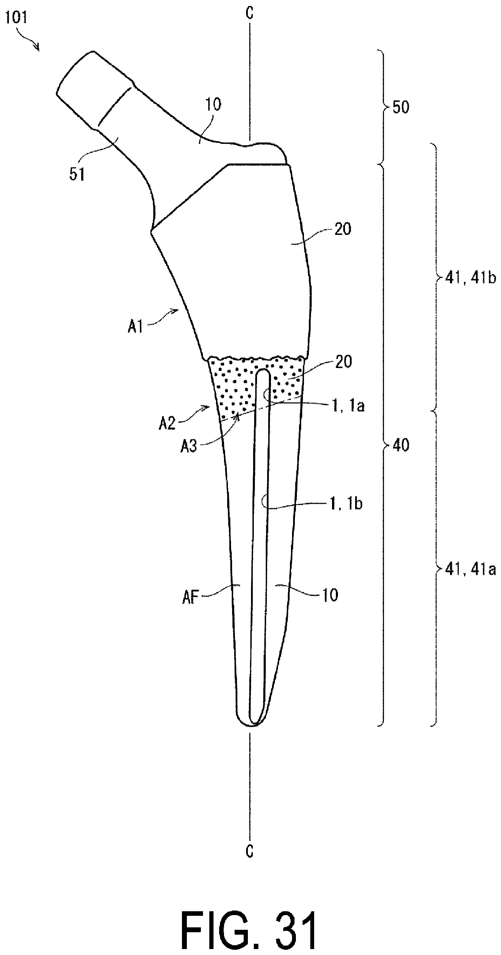

FIG. 31 illustrates an example different from that in FIG. 16. In the artificial joint stem 101 illustrated in FIG. 31, the end portion of the second region A2 on the distal side is a region extending along an oblique direction with respect to the center axis C extending along the groove 1.

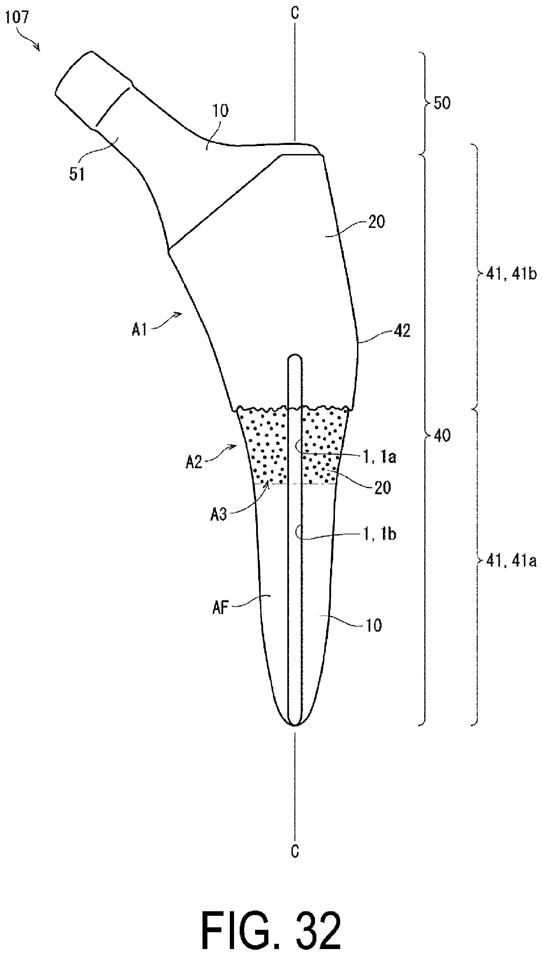

The artificial joint stem according to the present disclosure also includes an artificial joint stem 107 illustrated in FIG. 32. For example, the body portion 41 may include a distal portion 41a having the center axis C extending along the groove 1, and a proximal portion 41b extending toward the proximal side continuously from the distal portion 41a and shaped to curve such that the center of the proximal portion 41b departs further away from the center axis C as the proximal portion 41b extends toward the proximal side. The proximal portion 41b includes an end surface on the proximal side disposed away from the center axis C, and the neck portion 51 may be connected to the end surface on the proximal side.

The proximal portion 41b may be shaped to be farther away from the center axis C and then be closer to the center axis C as the proximal portion 41b extends from the distal side toward the proximal side on the side opposite to the side to which the neck portion 51 is connected. That is, the proximal portion 41b may have a protruding portion 42 on the side opposite to the side to which the neck portion 51 is connected. The second region A2 may be located on the distal side with respect to the protruding portion 42.

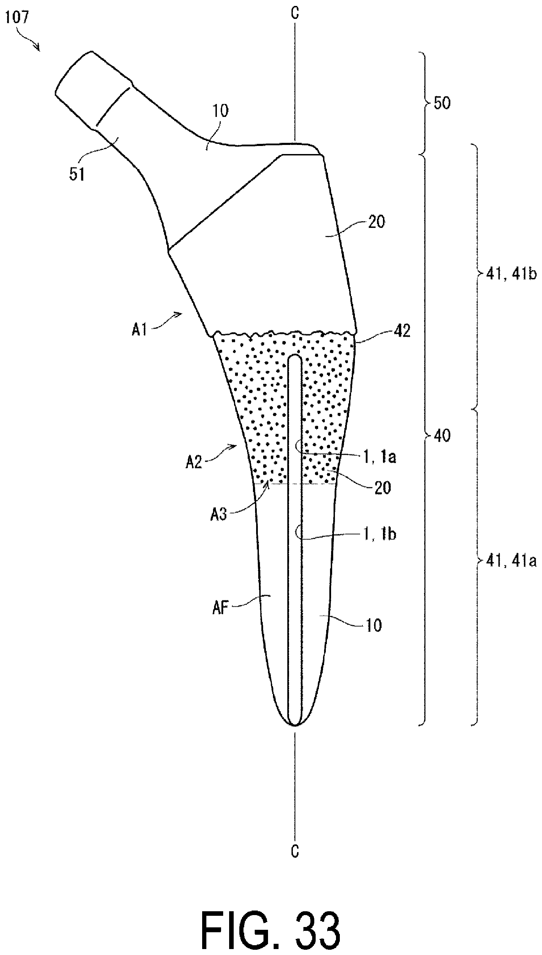

FIG. 33 illustrates an example different from that in FIG. 32. In the artificial joint stem 107 illustrated in FIG. 33, the end portion on the proximal side in the second region A2 is on the proximal side with respect to the protruding portion 42.

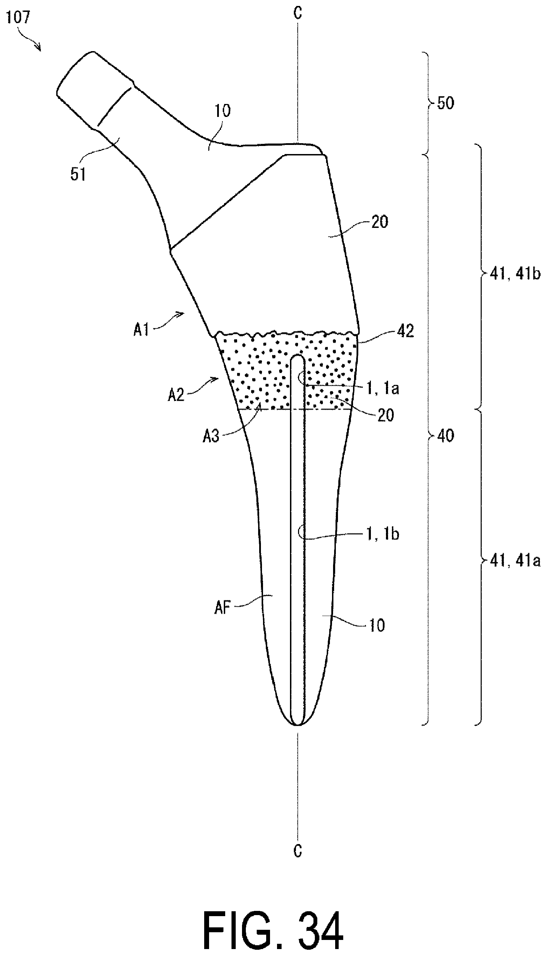

FIG. 34 illustrates an example different from that in FIG. 32. In the artificial joint stem 107 illustrated in FIG. 34, the end portion on the proximal side in the second region A2 is on the proximal side with respect to the protruding portion 42. The end portion on the distal side in the second region A2 is on the proximal side with respect to the end portion on the distal side in the second region A2 illustrated in FIG. 32, and is on the distal side with respect to the end portion on the proximal side of the groove 1.

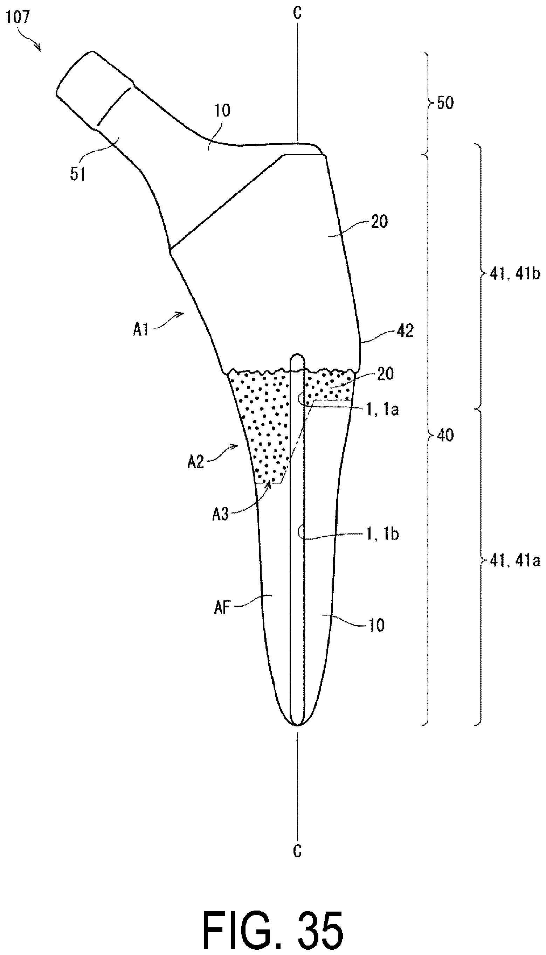

FIG. 35 illustrates an example different from that in FIG. 32. In the artificial joint stem 107 illustrated in FIG. 35, the second region A2 is located on the distal side with respect to the protruding portion 42. The end portion on the distal side in the second region A2 is stepped, and is located on the distal side on the side to which the neck portion 51 is connected with respect to the opposite side.

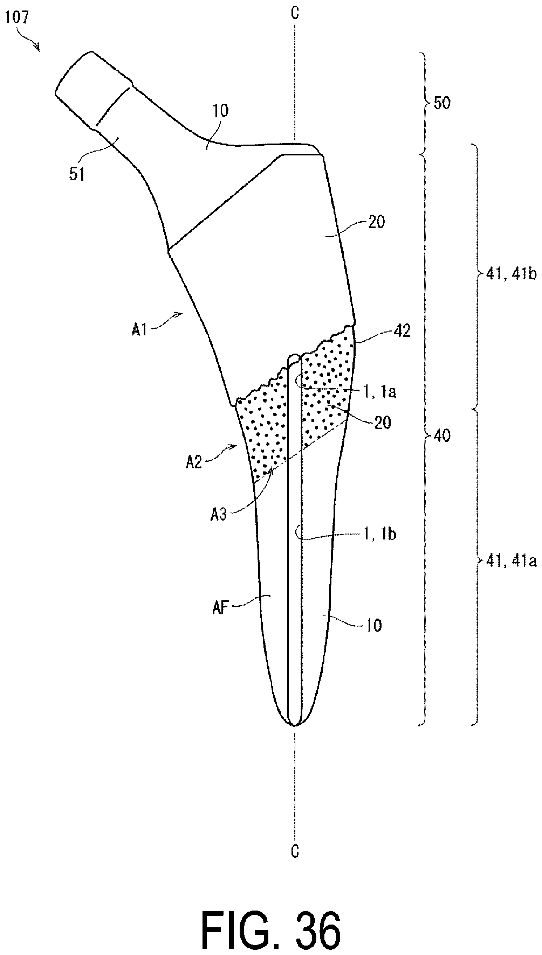

FIG. 36 illustrates an example different from that in FIG. 32. In the artificial joint stem 107 illustrated in FIG. 36, the second region A2 is a region extending along an oblique direction with respect to the center axis C extending along the groove 1. The protruding portion 42 is present in the second region A2.

### 2. Method of Manufacturing Artificial Joint Stem

In an embodiment, a method of manufacturing an artificial joint stem includes, for example, a preparation step, a groove forming step, a mirror-finishing step, and a coating film forming step. In the preparation step, a base 10 having a surface including a first region and a distal region is prepared. The groove forming step is a step of forming one or more grooves in the surface of the base 10. The mirror-finishing step is a step of finishing the surface of the base 10 like a mirror surface. The coating film forming step is a step of forming a coating film 20 on a part (the first region) of the surface of the base 10. The coating film forming step may be a step of forming a second coating film 30 containing a calcium phosphate-based material and an antimicrobial material so as to cover the first coating film 20.

In the preparation step, the base 10 can be prepared by molding a metal material into a desired shape by using a metal mold, an additive manufacturing method, or the like.

In the groove forming step, the groove can be formed by at least one selected from the group consisting of a cutting method, a rolling processing method, and a pressing method, for example. Note that, in the present embodiment, the groove is formed by milling, which is a type of the cutting method, for example. When the artificial joint stem includes the recess described above, a recess forming step may be only required to be provided in place of the groove forming step. The recess forming step uses a method similar to the groove forming step, for example.

In the mirror-finishing step, the surface of the base 10 can be mirror-finished by at least one selected from the group consisting of a fine cutting method, a fine grinding method, and a fine polishing process, for example. In the present embodiment, the surface of the base 10 is mirror-finished by performing barrel polishing, which is a type of the fine polishing method, for example.

In the mirror-finishing step, the entire surface of the base 10 may be mirror-finished. In the mirror-finishing step, a region of the surface of the base 10 other than the first region A1 may be mirror-finished. In the mirror-finishing step, only the distal region AF of the surface of the base 10 may be mirror-finished. By mirror-finishing a region of the surface of the base 10 other than the first region A1, the surface roughness of the first region A1 can be made higher than the surface roughness of the region other than the first region A1. A mirror-finished surface is expected to reduce biofilm formation compared to a non-mirror-finished surface.

Alternatively, in the mirror-finishing step, the entire surface of the base 10 may not be mirror-finished. In the mirror-finishing step, the distal region AF may not be mirror-finished. In the mirror-finishing step, the groove 1 may be mirror-finished. In the mirror-finishing step, only the groove 1 may be mirror-finished without mirror-finishing the entire surface of the base 10. In the mirror-finishing step, a region of the surface of the base 10 other than the groove 1 may be mirror-finished.

After the groove forming step and the mirror-finishing step, the coating film forming step can be performed. For example, the first coating film 20 can be formed by a thermal spraying method such as flame spraying, arc spraying, shield arc spraying, high-speed flame spraying, and plasma spraying, for example. For example, the first coating film 20 can be formed by physical vapor deposition or chemical vapor deposition such as sputtering, ion plating, ion beam deposition, and ion mixing. For example, the first coating film 20 can be formed by a wet coating method such as a sol-gel method. In the present embodiment, the first coating film 20 is formed by flame spraying, which is a type of the thermal spraying method, for example. The material constituting the coating film is also referred to as a coating material.

A first protective material may be used to form the first coating film 20 only in the first region A1. In this case, the coating film forming step may be preceded by a step of disposing the first protective material at a position to protect a region other than the first region while exposing the first region so that the coating film is not formed in the region other than the first region.

For example, a masking tape or a screen may be used as the first protective material. Alternatively, for example, a jig covering the base 10 may be used as the first protective material. Examples of the material of the first protective material include metals, glasses, resins, and composite materials thereof. Note that the first protective material may be in contact with the base 10, or may not be in contact with the base 10. When a masking tape is used as the first protective material, for example, the first protective material may be disposed on the region other than the first region. When a jig covering the base 10 is used as the first protective material, for example, the shape of the jig is not particularly limited, but may be, for example, tubular. The cross section of the tubular jig may be polygonal. The cross section of the tubular jig may be circular.

When a screen is to be disposed, the first coating film 20 can be formed in a specific region by placing the screen in a predetermined location. When a jig is used, the first coating film 20 can be formed in a specific region by placing the jig in a predetermined location.

In this case, for example, the first coating film 20 can be selectively formed only in a desired region by adjusting the positional relationship between the screen and a discharge nozzle configured to discharge the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the first coating film 20 may be formed while the base 10, the screen, and the discharge nozzle are fixed, or the first coating film 20 may be formed while moving at least one selected from the group consisting of the aforementioned. The angle of the discharge nozzle may be fixed or the first coating film 20 may be formed while changing the angle.

The first coating film 20 can be formed only in a desired region without using a protective material. For example, the coating film 20 can be selectively formed only in a desired region by adjusting the shape, angle, position, or the like of the discharge nozzle configured to discharge the discharge material.

For example, the discharge material may be discharged with the discharge nozzle located above the surface of the desired region. In this case, the coating film 20 may be formed by fixing the base 10 and moving the position and angle of the discharge nozzle, or the coating film 20 may be formed by fixing the discharge nozzle and moving the position and angle of the base 10. The discharge nozzle may be moved at a constant speed or at a variable speed. By changing the speed, the first coating film 20 can be formed in a desired region with a desired thickness. The discharge direction of the discharge material may be an angle of 90° or an angle of less than 90° with the vector extending from the tip of the discharge nozzle toward the base 10 or the surface of the rough surface, which are located at the shortest distance from the tip of the discharge nozzle.

A roughening step may be performed before the coating film forming step. The roughening step is a step of forming a rough surface in a roughening region of the first region A1 of the base 10. In the roughening step, a rough surface can be formed by at least one selected from the group consisting of a thermal spraying method, an additive manufacturing method, a chemical etching method, and a blasting method. Compared with the blasting method, the thermal spraying method, the additive manufacturing method, or the chemical etching method can increase the surface roughness.

The material discharged toward the base 10 in the thermal spraying method is referred to as a thermal spraying material. The material discharged toward the base 10 in the additive manufacturing method is referred to as an additive manufacturing material. The material discharged toward the base 10 when processing by the chemical etching method is performed is referred to as a chemical etching material. The material discharged toward the base 10 when processing by the blasting method is performed is referred to as a blasting material. As the thermal spraying material, the materials exemplified as the material of the base 10 can be used. As the additive manufacturing material, the materials exemplified as the material of the base 10 can be used. The first coating film 20 described above may be formed by at least one of the thermal spraying method and the additive manufacturing method. Examples of the chemical etching method include alkali treatment. Examples of the blasting method include sandblasting.

A second protective material may be used to form a rough surface only in a desired region. In this case, the roughening step may be preceded by a step of disposing the second protective material so as to protect a region other than a roughening region while exposing the roughening region so that the rough surface is not formed in the region other than the roughening region. Note that the roughening region may be located inside the first region A1 or may be located in the first region A1. That is, the second protective material may be disposed so as to protect a part of the first region A1 or a region other than the first region A1.

For example, a masking tape or a screen may be used as the second protective material. Alternatively, for example, a jig covering the base 10 may be used as the second protective material. Examples of the material of the second protective material include metals, glasses, resins, and composite materials thereof. Note that the second protective material may be in contact with the base 10, or may not be in contact with the base 10. When a jig covering the base 10 is used as the second protective material, for example, the shape of the jig is not particularly limited, but may be, for example, tubular. The cross section of the tubular jig may be polygonal. The cross section of the tubular jig may be circular.

When a screen is to be placed, a rough surface can be formed in a specific region by placing the screen in a predetermined position. When a jig is to be used, a rough surface can be formed in a specific region by placing the jig in a predetermined position.

In this case, for example, the rough surface can be selectively formed only in a desired region by adjusting the positional relationship between the screen and a discharge nozzle for discharging the thermal spraying material, the additive manufacturing material, the chemical etching material, the blasting material, or the coating material. In this case, a tip of the discharge nozzle is only required to be disposed, for example, in a straight line with the surface of the desired region without being separated by the screen. Hereinafter, a thermal spraying material, an additive manufacturing material, a chemical etching material, a blasting material, or a coating material discharged from the discharge nozzle is also referred to as a discharge material. Without being limited to the above, the rough surface may be formed while the base 10, the screen, and the discharge nozzle are fixed, or the rough surface may be formed while moving at least one selected from the group consisting of the aforementioned. The angle of the discharge nozzle may be fixed or the rough surface may be formed while changing the angle. Note that a rough surface may be formed only in a desired region without using a protective material.

As described above, when the artificial joint stem of the present disclosure includes a rough surface, for example, in the roughening step, while exposing a part of the surface of the base 10, the second protective material may be disposed on the base 10 so as to protect another part of the base 10, so that a rough surface may be formed on the exposed part of the surface. The manufacturing method according to the present disclosure may further include a step of removing the second protective material after the roughening step and before the coating film forming step.

After the second protective material is removed, a step of scraping the edge of the rough surface, for example, the edge of the first coating film 20, may be performed. Thus, the concentration of stress at the edge of the first coating film 20 can be avoided, and irritation to the living tissue can be reduced.

A second masking tape may be used as the second protective material. In this case, the manufacturing method according to the present disclosure may further include a step of, while exposing a part of the surface of the base 10, attaching the second masking tape to another part of the base 10, before the roughening step.

In the coating film forming step, while exposing a part of the surface of the base 10, the first protective material may be disposed on the base 10 so as to protect another part of the base 10, so that the first coating film 20 may be formed on the exposed part of the surface. The first protective material can be removed after the coating film forming step. Here, a first masking tape may be used as the first protective material. In this case, the manufacturing method according to the present disclosure may further include a step of, while exposing a part of the surface of the base 10, attaching the first masking tape to another part of the base 10, before the coating film forming step.

As the material of the second protective material, a material having higher thermal resistance than that of the first protective material can be used. For example, a material which is not melted or pyrolyzed for 1 minute under a thermal spraying condition of 8000°C may be used as the material of the second protective material, and a material which is not melted or pyrolyzed for 1 minute under a thermal spraying condition of 3000°C may be used as the material of the first protective material. A specific example of such materials includes a composite material of glass and resin.

When processing by the blasting method is performed in the roughening step, a material which is not melted or pyrolyzed at room temperature may be used as the material of the second protective material. A specific example of such a material includes a resin.

In the manufacturing method according to the present disclosure, in the step of forming the rough surface or the first coating film 20, a protective material may be disposed in addition to the first protective material and the second protective material described above. For example, in the step of forming the rough surface or the coating film 20, a protective material may be disposed on a part or all of the exposed portion 50. Thus, the presence or absence of the formation of the rough surface or the first coating film 20 in the exposed portion 50 can be appropriately controlled. For example, by disposing a protective material in a region of the exposed portion 50 on the side far from the embedded portion 40 and forming the first coating film 20 in the exposed region, the first coating film 20 can be formed on a region of the exposed portion 50 on the side close to the embedded portion 40.

In summary, each step can be performed in the order illustrated in FIG. 14, for example. FIG. 14 is a flowchart illustrating a method of manufacturing an artificial joint stem 100 according to an embodiment. First, a second protective material is disposed, and after a roughening step is performed, the second protective material can be removed. Thereafter, a groove forming step can be performed, and a mirror-finishing step can be performed. Then, a first protective material is disposed, and then a coating film forming step can be performed.

The manufacturing method according to the present disclosure may or may not include a cleaning step between the steps. For example, the manufacturing method according to the present disclosure may include a step of cleaning the base 10, or the base 10 and the first coating film 20, after the roughening step. The cleaning method is not particularly limited. The cleaning method may be, for example, a method of immersing in a liquid such as water or an organic solvent such as alcohol, or a method of showering using the liquid. Alternatively, the cleaning method may be a method of blowing a gas such as air, nitrogen, or argon. Through the cleaning step, the excess thermal spraying material or the like and/or scrapes or the like generated by the roughening step can be removed.

Although the manufacturing method of the artificial joint stem 100 including the groove has been described above, the manufacturing method according to the present disclosure is not particularly limited to the steps described above. For example, in the manufacturing method described above, an example has been described where after the roughening step of roughening the surface of the base 10, the groove forming step of forming a groove is performed, and then the coating film forming step of forming a coating film is performed. Instead, after the groove forming step, the roughening step may be performed, and then the coating film forming step may be performed.

The roughening step may include, in order, a first roughening step of forming a first rough surface by a thermal spraying method and a second roughening step of forming a second rough surface by a chemical etching method or a blasting method. Here, a region where the first rough surface is formed by the thermal spraying method is referred to as a first roughening region, a region where the second rough surface is formed by the chemical etching method or blasting method is referred to as a second roughening region, and a region where no rough surface is formed is referred to as a non-roughening region.

In the first roughening step, a third protective material (the second protective material described above) may be disposed on the base 10 so as to protect the second roughening region and the non-roughening region while exposing the first roughening region, thereby forming the first rough surface in the exposed first roughening region. The manufacturing method according to the present disclosure may further include a step of removing the third protective material after the first roughening step and before the second roughening step. In the second roughening step, a fourth protective material may be disposed on the base 10 so as to protect the non-roughening region while exposing the second roughening region, thereby forming the second rough surface in the exposed second roughening region. The second roughening step may be performed such that the surface of the second rough surface formed in the second roughening step has a surface roughness lower than the surface of the first rough surface in the first roughening region. The manufacturing method according to the present disclosure may include a step of removing the fourth protective material after the second roughening step and before the coating film forming step.

For example, a fourth masking tape may be used as the fourth protective material. In this case, the manufacturing method according to the present disclosure may further include a step of attaching a fourth masking tape to the non-roughening region while exposing the first roughening region and the second roughening region before the second roughening step. As the material of the fourth protective material, a material having lower thermal resistance than the above-described third protective material may be used. For example, a material which is not melted or pyrolyzed at room temperature may be used as the material of the fourth protective material. Specifically, a resin may be used as the material of the fourth protective material.

In the second roughening step, the non-roughening region may or may not be covered with a protective material. The first roughening region and the non-roughening region may be protected, and only the second roughening region may be processed by at least one selected from the group consisting of a chemical etching method and a blasting method. Alternatively, a fourth protective material may be disposed so as to expose the first roughening region, and the rough surface of the exposed first roughening region and the second roughening region may be processed by at least one selected from the group consisting of a chemical etching method and a blasting method. Thus, an excess thermal spraying material or the like remaining on the rough surface of the first roughening region can be removed, and a rough surface can be also formed in the second roughening region.

The method of manufacturing the artificial joint stem 100 may initially include a step of preparing a base 10 in which the first roughening region, the second roughening region, and the non-roughening region are located in this order.

### 3. Use of Artificial Joint Stem

Although the artificial joint stem 100 illustrated in FIG. 1 has a shape mainly assuming a stem for an artificial hip joint, artificial joints to which the artificial joint stem according to the present disclosure is applied are not limited to artificial hip joints. Examples of the artificial joint include an artificial hip joint, an artificial knee joint, an artificial ankle joint, an artificial shoulder joint, an artificial elbow joint, and an artificial finger joint.

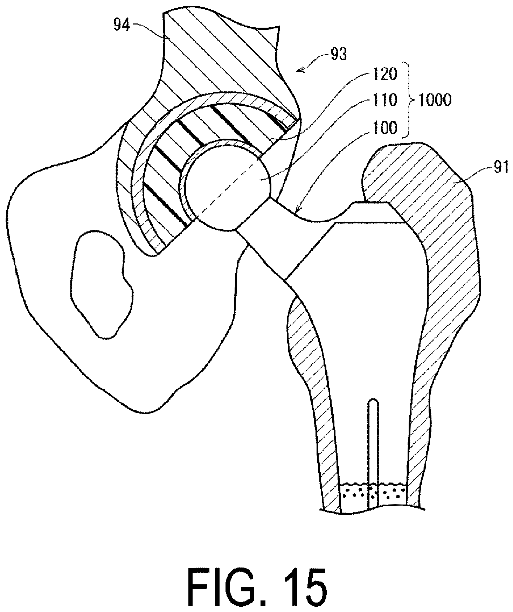

Referring now to FIG. 15, an example in which the artificial joint stem 100 is used as a part of an artificial hip joint 1000 will be described below. The artificial hip joint 1000 may include a bone head 110 and an acetabular cup 120 in addition to the artificial joint stem 100. The bone head 110 and the acetabular cup 120 may be formed of the same material as the base 10 of the artificial joint stem 100. The bone head 110 and the acetabular cup 120 may be formed of a different material from the base 10 of the artificial joint stem 100. The artificial joint stem 100 is embedded in a femur 91. The bone head 110 is disposed at the exposed portion 50 of the artificial joint stem 100. The acetabular cup 120 is fixed to an acetabular 94 of a hip bone 93. The acetabular cup functions as a hip joint by fitting and sliding the bone head 110 into the depression of the acetabular cup 120.

The invention according to the present disclosure has been described above based on various drawings and embodiments. However, the invention according to the present disclosure is not limited to the embodiments described above. That is, the invention according to the present disclosure can be modified in various ways within the scope described in the present disclosure, and embodiments obtained by combining technical means disclosed in the different embodiments as appropriate are also included in the technical scope of the invention according to the present disclosure. In other words, note that a person skilled in the art can easily make various variations or modifications based on the present disclosure. Note that these variations or modifications are included in the scope of the present disclosure.

### REFERENCE SIGNS

1 Groove
10 Base
20 First coating film
21 Thermal spraying layer
30 Second coating film
40 Embedded portion
41 Body portion
50 Exposed portion
51 Neck portion
100 Artificial joint stem
1000 Artificial hip joint

## Claims

1. An artificial joint stem comprising:
a base having a surface; and
a first coating film located in a first region on the surface of the base, wherein
the first region comprises a second region having a portion where the first coating film is not located; and
the second region comprises a third region in which the first coating film is located and around which the first coating film is not located.

2. The artificial joint stem according to claim 1, wherein
in a region of the first region adjacent to the second region, a thickness of the first coating film decreases toward the second region.

3. The artificial joint stem according to claim 1 or 2, wherein
when a side embedded in a bone when the artificial joint stem is used is defined as a distal side and an opposite side is defined as a proximal side, the second region is a region at an end portion on the proximal side or an end portion on the distal side in the first region.

4. The artificial joint stem according to claim 3, wherein
the second region is a region at the end portion on the distal side in the first region.

5. The artificial joint stem according to any one of claims 1 to 4, wherein
the second region comprises a fourth region in which the first coating film is not located and around which the first coating film is located.

6. The artificial joint stem according to any one of claims 1 to 5, wherein
the base has a groove; and
the first region in which the first coating film is disposed comprises at least a part of the groove.

7. The artificial joint stem according to claim 6, wherein
for an inside portion of the groove and an outside portion of the groove adjacent to each other in the first region, a thickness of the first coating film in the inside portion of the groove is greater than a thickness of the first coating film in the outside portion of the groove.

8. The artificial joint stem according to claim 6 or 7, wherein
a thickness of the first coating film in the inside portion of the groove increases toward a depth direction of the groove.

9. The artificial joint stem according to any one of claims 6 to 8, wherein
for an inside portion of the groove and an outside portion of the groove adjacent to each other in the first region, a surface roughness of the first coating film in the inside portion of the groove is higher than a surface roughness of the first coating film in the outside portion of the groove.

10. The artificial joint stem according to any one of claims 6 to 9, wherein
when a side embedded in a bone when the artificial joint stem is used is defined as a distal side and an opposite side is defined as a proximal side, a thickness of the first coating film in an inside portion of the groove decreases from the proximal side toward the distal side.

11. The artificial joint stem according to any one of claims 6 to 10, wherein
in the second region, a ratio of a surface area of a region where the first coating film is not located to a surface area of an inside portion of the groove is less than a ratio of the surface area of the region where the first coating film is not located to a surface area of an outside portion of the groove.

12. The artificial joint stem according to any one of claims 1 to 11, wherein
the base has a groove; and
for an inside portion of the groove and an outside portion of the groove adjacent to each other, a surface roughness of the inside portion of the groove is different from a surface roughness of the outside portion of the groove.

13. The artificial joint stem according to claim 12, wherein
the surface roughness of the inside portion of the groove is higher than the surface roughness of the outside portion of the groove.

14. The artificial joint stem according to claim 12, wherein
the surface roughness of the inside portion of the groove is lower than the surface roughness of the outside portion of the groove.

15. The artificial joint stem according to any one of claims 12 to 14, wherein
the surface roughness of the inside portion of the groove increases toward a depth direction of the groove.

16. The artificial joint stem according to any one of claims 1 to 15, further comprising:
a second coating film located on the first region where the first coating film is located, wherein
the second coating film contains a calcium phosphate-based material.
